# EUROPEAN PATENT APPLICATION

(11) **EP 4 840 433 A1**
(43) Date of publication of application: **18.11.2026**
(21) Application number: 25201738.9
(22) Date of filing: 11.09.2025
(51) Int. Cl.: A61F 13/494

(54) **ABSORBENT ARTICLE WITH NOVEL BARRIER CUFFS**

(30) Priority: 16.05.2025 EP 25176968; 21.05.2025 EP 25178028
(71) Applicant: Ontex BV, 9255 Buggenhout (BE); Ontex Group NV, 9320 Erembodegem (BE)
(72) Inventor: Müller, Andreas, 56727 Mayen (DE); Lambertz, Christina, 56566 Neuwied (DE)
(74) Representative: Macchetta, Andrea

(57) **Abstract**

An absorbent article, such as a diaper, includes a liquid permeable topsheet, a liquid impermeable backsheet, and an absorbent core. A pair of barrier leg cuffs are included, each comprising an inner cuff element. The inner cuff element comprises an inner folded edge and a plurality of inner elastic elements disposed proximate the inner folded edge. The inner cuff element is substantially T-shaped and comprises a cutaneous sealing strut and an upstanding connecting strut adjoining the cutaneous sealing strut, wherein the upstanding connecting strut is formed by a nonwoven layer, and wherein the cutaneous sealing strut comprises the same nonwoven layer.

## Description

### TECHNICAL FIELD

The present disclosure pertains to the technical field of absorbent hygiene articles such as disposable diapers or (diaper) pants, disposable incontinence diapers or pants, pads, provided with barrier cuffs reducing the risk of side leakage and offering comfort.

### BACKGROUND

Absorbent articles for personal hygiene are designed to absorb and contain bodily exudates, such as a large quantity of urine. Side leakage is a common problem for absorbent articles. Gushes of urine discharge can temporarily flood the surface of the absorbent article, leaking over the side margin.

To adress this problem, elastic gathering elements have been incorporated in various ways along the lateral edges of the absorbent article. These may include barrier cuffs which are elasticized flaps which stand substantially upright, more preferably inwardly towards the longitudinal centerline of the article, at least within the crotch region. Typically, said barrier cuffs include at least one elastic that is connected primarily at its opposing ends to the diaper. When the absorbent article is worn, typically, the uprising barrier cuffs extend through the groin area and along both of the buttocks of the wearer. The elastic gathering elements may also include gasketing cuffs, also called leg elastics, which are elasticized flaps which do not stand substantially upright, and which are more preferably disposed outwardly towards the longitudinal side edges of the article, within the crotch region. Typically, said gasketing cuffs include at least one elastic that is connected substantially throughout its length to the diaper (e.g., multiple bonds along length of elastic to create gathers). When the absorbent article is worn, typically, the gasketing cuffs will encircle the thighs and create a gasketing action against the thighs.

WO202408297, for example, describes an absorbent article having a nonwoven outer cover layer which overlies the backsheet and forms an outer surface of the absorbent article and which has side portions which extend laterally outside the backsheet and are formed into leg cuff folds, the leg cuff folds being elasticated by a plurality of spaced-apart elastic elements. A pair of nonwoven barrier cuff strips are arranged with a standing gather portion of each barrier cuff strip forming part of a standing gather, the barrier cuff strip being attached to the topsheet along a standing gather hinge line and having one or more pretensioned elastic elements arranged along a free standing gather edge. A pair of leg cuffs are arranged outward of the standing gathers, each leg cuff comprising the folded-back, elasticated side portion of the outer cover layer and a leg cuff portion of the corresponding nonwoven barrier strip. The leg cuff portion of the nonwoven barrier strip overlies at least one elastic element of the plurality of elastic elements. A drawback with this absorbent article is that it may be less skin-friendly and comfortable to wear as all body-contacting surfaces of the article are not constituted by soft nonwoven materials and/or the presence of a large number of elastics at a leg edge my cause pressure and tension build-up that is more easily prone to skin irritation and pressure marks. Moreover, such execution is complex and costly to attain.

Although barrier cuffs are today very efficient in reducing the risk of side leakage, we have noted that in certain circumstances, liquid could still pass through this barrier and cause leakage towards the longitudinal sides of absorbing articles.

The present disclosure aims to provide a novel absorbent article particularly designed to even more reduce the risks of side leakage, whilst not compromising on other aspects like absorbency and comfort.

### SUMMARY

In one aspect, the present disclosure relates to an absorbent article comprising a liquid permeable topsheet, a liquid impermeable backsheet, and an absorbent core disposed at least partially between the topsheet and the backsheet. A pair of barrier leg cuffs are included, each comprising an inner cuff element, wherein the inner cuff element comprises an inner folded edge and a plurality of inner elastic elements disposed proximate the inner folded edge, the inner cuff element is substantially T-shaped, and comprises a cutaneous sealing strut and an upstanding connecting strut adjoining said cutaneous sealing strut, wherein the upstanding connecting strut is formed of a nonwoven layer, and wherein said cutaneous sealing strut comprises the same said nonwoven layer. This T-shaped configuration may, combined with the cutaneous sealing strut and nonwoven-formed upstanding connecting strut, provide a superior combination of effective fluid containment and gentle contact with the skin as will be described in more detail herein.

According to a preferred embodiment of the disclosure, an absorbent article has each barrier leg cuff formed from a single web of material and comprising an inner cuff element and an outer cuff element, and the outer cuff element comprises an outer folded edge and from 1 to 3 outer elastic elements, wherein the outer elastic elements are disposed proximate the outer folded edge, and one or more leg elastics are disposed at least partly between each of the pair of barrier leg cuffs and at least one of the topsheet and the backsheet.

According to a preferred embodiment of the disclosure, the outer elastic elements are encapsulated by the outer cuff element within an outer fold thereof.

According to a preferred embodiment of the disclosure, the one or more leg elastics are disposed at least partly between each of the pair of barrier leg cuffs and the backsheet.

According to a preferred embodiment of the disclosure, the article comprises at least 2 of said leg elastics, preferably from 2 to 4 of said leg elastics.

According to a preferred embodiment of the disclosure, the inner cuff element forms an up-standing gather.

According to a preferred embodiment of the disclosure, the outer cuff element of each of the pair of barrier leg cuffs is positioned at a lateral edge of said article, said lateral edge extending substantially parallel to a longitudinal axis and preferably wherein a pair of said lateral edges together with a pair of oppositely disposed transversal edges extending substantially perpendicular to the longitudinal axis form a perimeter of a chassis of said article.

According to a preferred embodiment of the disclosure, the inner cuff element comprises at least 2, preferably at least 3, preferably from 3 to 5, inner elastic elements. This may enhance the sealing effect of the inner cuff.

According to a preferred embodiment of the disclosure, the inner cuff element comprises a skin-contact surface having a width when viewed from a section taken about an axis being substantially perpendicular to the longitudinal axis, and wherein said width is greater than a diameter Ø of inner elastic elements; preferably wherein said width is more than 2Ø, more preferably from 3Ø to 150, even more preferably from 4Ø to 10Ø, such width as described herein generally extends in a direction along said axis being substantially perpendicular to the longitudinal axis. This may ensure that the elastic elements do not directly contact the skin, improving comfort.

According to a preferred embodiment of the disclosure, the cutaneous sealing strut comprises at least two nonwoven layers and having said inner elastic elements disposed therebetween and/or encapsulated therein, preferably said at least two nonwoven layers being formed by a single nonwoven layer folded around said inner elastic elements.

According to a preferred embodiment of the disclosure, the cutaneous sealing strut comprises one or more adhesive-free gaps between neighbouring inner elastic elements preferably such that the at least two nonwoven layers are not joined to each other at positions(s) corresponding to said gap(s) and are able to stay apart.

According to a preferred embodiment of the disclosure, the inner elastic elements are strand-coated with adhesive. This may ensure good adhesion of the elastic elements to the nonwoven layers whilst achieving optimal softness. Alternatively, the elastics can be applied to a nonwoven surface that comprises continuous or discontinuous stripes of adhesive thereon.

According to a preferred embodiment of the disclosure, the cutaneous sealing strut comprises one or more macro-wrinkles, each macro-wrinkle preferably substantially corresponding to a position of the one or more adhesive-free gaps.

According to a preferred embodiment of the disclosure, the pair of barrier leg cuffs comprise a hydrophobic nonwoven, preferably wherein the hydrophobic nonwoven comprises a spunbond nonwoven, even more preferably wherein the spunbond nonwoven is a lofty spunbond nonwoven comprising crimped fibers.

According to a preferred embodiment of the disclosure, the one or more leg elastics are disposed directly between each of the pair of barrier leg cuffs and at least one of the topsheet and the backsheet, preferably wherein the one or more leg elastics are disposed directly between each of the pair of barrier leg cuffs and the backsheet

According to a preferred embodiment of the disclosure, the outer cuff element at least partly overlaps the backsheet on the skin-facing side of the backsheet, and preferably wherein at least a portion of the outer cuff element is disposed directly or indirectly, preferably directly, over the backsheet.

### BRIEF DESCRIPTION OF THE FIGURES

The disclosure can be better understood with reference to the following drawings and description. The components in the figures are not necessarily to scale, emphasis instead being placed upon illustrating various preferred embodiments of the disclosure. These embodiments are provided as non-limiting examples to aid in understanding the inventive concepts.

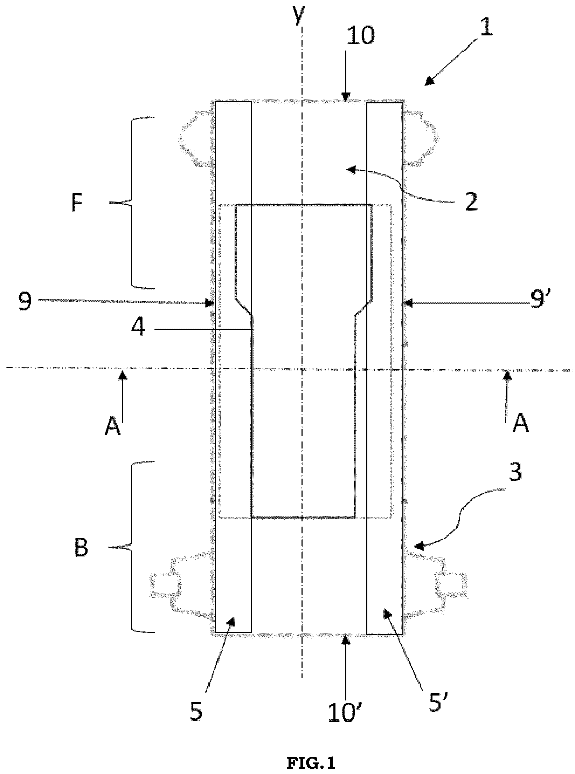

**Fig. 1** Top plan view of an absorbent article illustrating a general configuration according to an aspect of the disclosure. The absorbent article may be a diaper, an incontinence brief, or another similar absorbent product. The view shows the arrangement of the topsheet, backsheet, absorbent core, and barrier leg cuffs in one possible configuration.

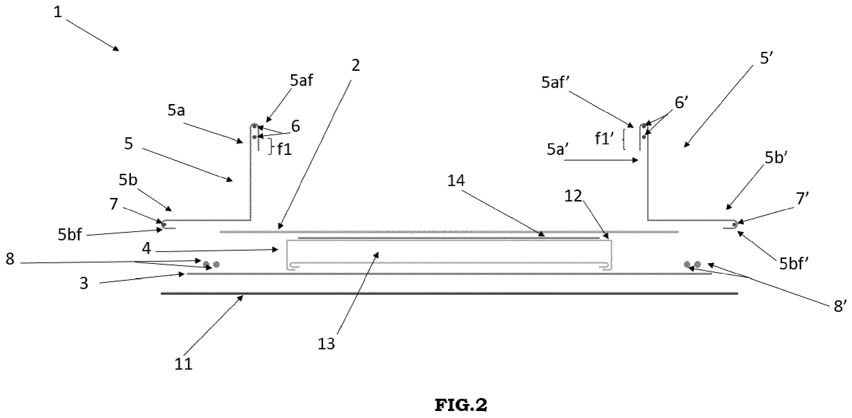

**Fig. 2** Section view of an absorbent article according to Fig. 1 about section A-A. This cross-sectional view provides a more detailed look at the layers and components of the absorbent article along the indicated section line. It illustrates a possible configuration of the topsheet, backsheet, absorbent core, and barrier leg cuffs, and elastic elements.

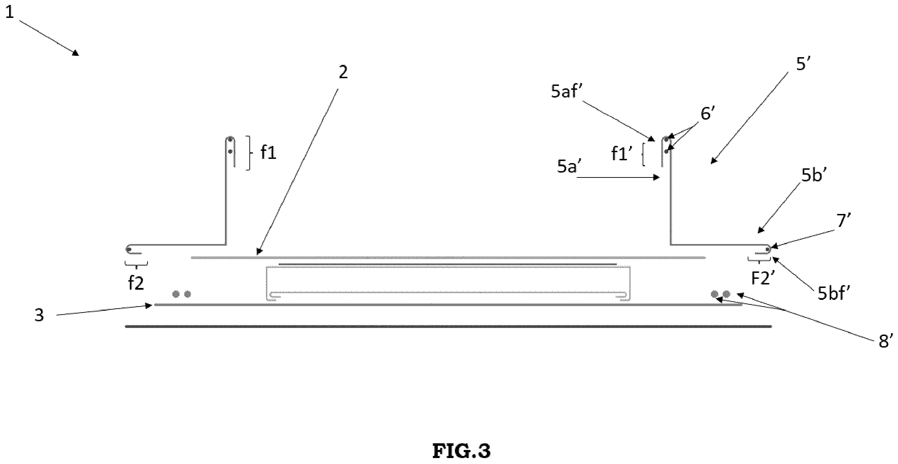

**Fig. 3** Section view identical to Fig.2 but with fewer components being called out by reference numerals to improve intelligibility.

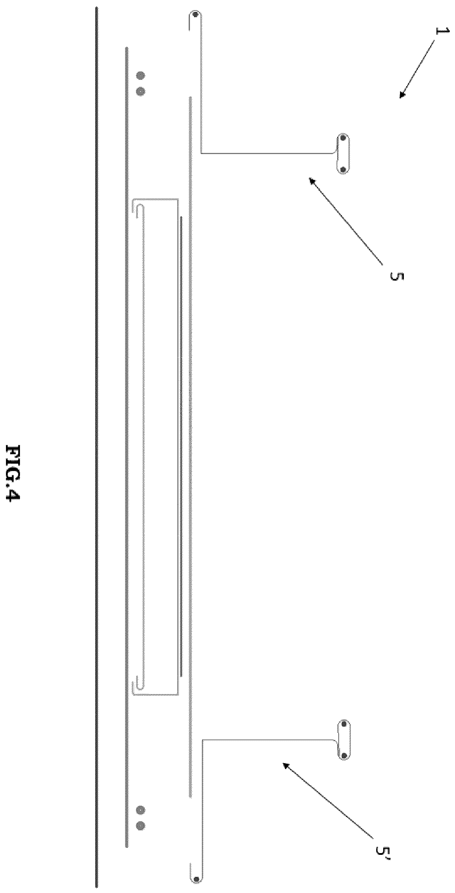

**Fig. 4** Section view identical to Fig.3 but with a T-shaped cuff as described according to embodiments herein.

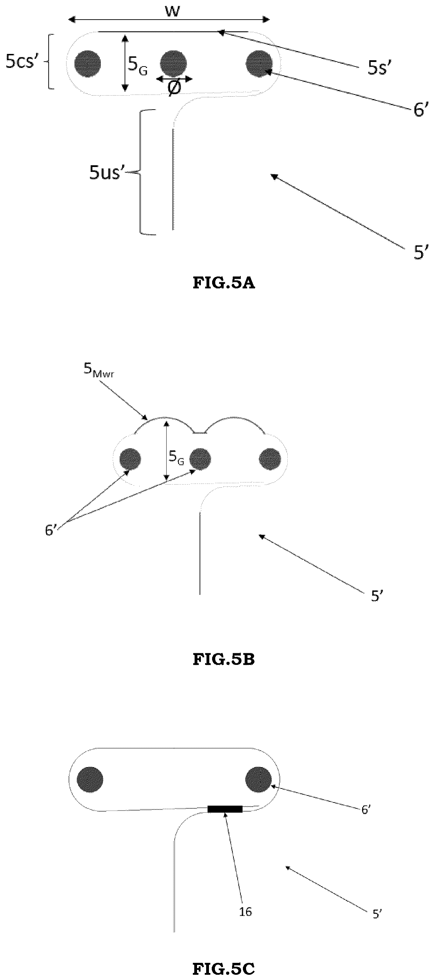

**Fig. 5A-C** Section views of a T-shaped cuff according to a preferred embodiment of the disclosure. These sectional views depict a possible T-shaped configuration for the inner cuff element of the barrier leg cuffs. Different arrangements of elastic elements and nonwoven layers are shown as non-limiting examples.

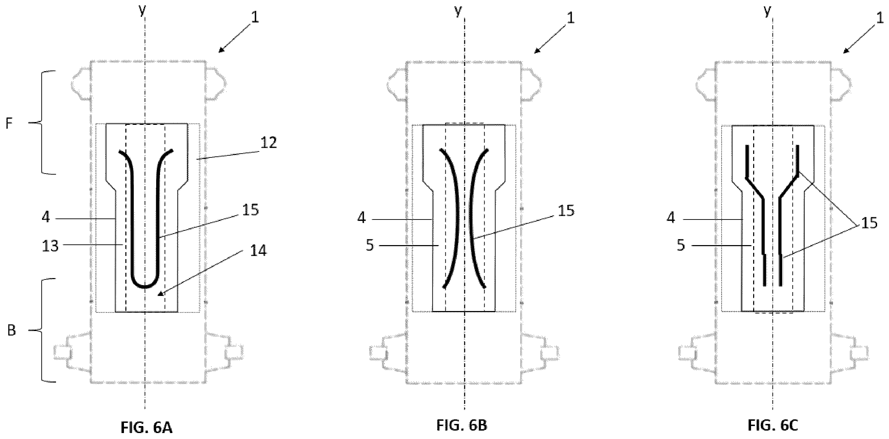

**Fig. 6A-C** Absorbent articles according to embodiments of the present disclosure comprising channels. These figures illustrate possible embodiments of the absorbent article incorporating channels within the absorbent core structure. The channels may vary in shape, size, and arrangement, and the figures provide non-limiting examples of such variations.

### DETAILED DESCRIPTION

Unless otherwise defined, all terms used in disclosing characteristics of the disclosure, including technical and scientific terms, have the meaning as commonly understood by one of ordinary skill in the art to which this disclosure belongs. By means of further guidance, term definitions are included to better appreciate the teaching of the present disclosure.

As used herein, the following terms have the following meanings:
"A", "an", and "the" as used herein refers to both singular and plural unless the context clearly dictates otherwise. By way of example, "a compartment" refers to one or more compartments.

"About" as used herein referring to a measurable value such as a parameter, an amount, a temporal duration, and the like, encompass variations of +/-20% or less, preferably +/-10% or less, more preferably +/-5% or less, even more preferably +/-1% or less, and still more preferably +/-0.1% or less of and from the specified value, in so far such variations are appropriate to perform in the disclosed invention. However, it is to be understood that the value to which the modifier "about" refers is itself also specifically disclosed.

"Comprise", "comprising", "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

The recitation of numerical ranges by endpoints includes all numbers and fractions subsumed within that range, as well as the recited endpoints.

"Absorbent article" refers to devices that absorb and contain liquid, and more specifically, refers to devices that are placed against or in proximity to the body of the wearer to absorb and contain the various exudates discharged from the body. Absorbent articles include but are not limited to diapers, adult incontinence briefs, training pants and diaper holders and liners, Absorbent articles preferably comprise a longitudinal axis and a transversal axis perpendicular to said longitudinal axis. The longitudinal axis is hereby conventionally chosen in the front-to-back direction of the article when referring to the article being worn, and the transversal axis is conventionally chosen in the left-to-right direction of the article when referring to the article being worn. The absorbent article includes a chassis. The absorbent article may include liquid wicking layers, liquid intake layers, liquid distribution layers, transfer layers, barrier layers, wrapping layers and the like, as well as combinations thereof. Disposable absorbent articles and the components thereof can operate to provide a skin-facing surface and a garment-facing surface.

A "chassis" of a disposable absorbent articles can include a liquid pervious topsheet, a backsheet joined to the top sheet, and an absorbent core positioned and held between the top sheet and the back sheet. The top sheet is operatively permeable to the liquids that are intended to be held or stored by the absorbent article, and the back sheet may or may not be substantially impervious or otherwise operatively impermeable to the intended liquids. The chassis comprises a front (F) waist portion or region, a back (B) waist portion or region, an intermediate crotch portion or region which interconnects the front and back waist portions. When used herein, reference to a "front" portion refers to that part of the chassis which is generally located on the front of a wearer when in use. Reference to the "back" portion refers to the portion of the chassis generally located at the back of the wearer when in use, and reference to the "crotch" portion refers to that portion which is generally located between the legs of a wearer when in use. The crotch portion is an area where repeated fluid surge typically occurs.

The "absorbent core" is the absorbent structure disposed between the topsheet and the backsheet of the chassis and is capable of absorbing and retaining liquid body exudates. The size and the absorbent capacity of the absorbent core should be compatible with the size of the intended wearer and the liquid loading imparted by the intended use of the chassis. Further, the size and the absorbent capacity of the absorbent core can be varied to accommodate wearers ranging from infants through adults. It may be manufactured in a wide variety of shapes (for example, rectangular, trapezoidal, T-shape, I-shape, hourglass shape, etc.) and from a wide variety of materials. Examples of commonly occurring absorbent materials are cellulosic fluff pulp, tissue layers, highly absorbent polymers (so called superabsorbent polymer particles (SAP)), absorbent foam materials, absorbent nonwoven materials or the like. It is common to combine cellulosic fluff pulp with superabsorbent polymers in an absorbent core. It can contain embossed channels, channels substantially free of material, channels with lower basis weight than the rest of the core, etc. The absorbent core is disposed between the topsheet and the backsheet; both the topsheet and the backsheet generally extend beyond the side edges of the absorbent core. The juxtaposed areas of the topsheet and the backsheet are typically secured together such as by adhesive.

"Acquisition and distribution layer", "ADL" or "surge management portion" refers to a sublayer which preferably is a nonwoven wicking layer under the top sheet of an absorbent product, which speeds up the transport and improves distribution of fluids throughout the absorbent core. The surge management portion is typically less hydrophilic than the retention portion, and has the ability to quickly collect and temporarily hold liquid surges, and to transport the liquid from its initial entrance point to other parts of the absorbent structure, particularly the retention portion. This configuration can help to prevent the liquid from pooling and collecting on the portion of the absorbent garment positioned against the wearer's skin, thereby reducing the feeling of wetness by the wearer. Preferably, the surge management portion is positioned between the top sheet and the retention portion.

The term "adhesive" as used herein is intended to refer to any suitable hot melt, water or solvent borne adhesive that can be applied to a surface in the required pattern or network of adhesive areas. Accordingly, suitable adhesives include conventional hot melt adhesives, pressure-sensitive adhesives and reactive adhesives (i.e., polyurethane).

"Bonded" refers to the joining, adhering, connecting, attaching, or the like, of at least two elements. Two elements will be considered to be bonded together when they are bonded directly to one another or indirectly to one another, such as when each is directly bonded to intermediate elements. "Join", "joining", "joined", or variations thereof and "attach", "attaching", "attached", or variations thereof, when used in describing the relationship between two or more elements, means that the elements can be connected together in any suitable manner, such as by heat sealing, ultrasonic bonding, thermal bonding, by adhesives, stitching, or the like. Further, the elements can be joined or attached together either directly or indirectly, i.e. they may have one or more elements interposed between them, all of which are connected together. For the sake of clarity, adhesive is not to be considered as an intervening element for the definition of direct or indirect attachment. When describing an attachment "without intervening layer of material" or "without any intervening material", this does not exclude the presence of adhesive.

As used herein, the term "adhesive bonding" means a bonding process which forms a bond by application of an adhesive. Such application of adhesive may be by various processes such as slot coating, spray coating and other topical applications. Further, such adhesive may be applied within a product component and then exposed to pressure such that contact of a second product component with the adhesive containing product component forms an adhesive bond between the two components.

As used therein, the term "associated" encompasses configurations in which two elements are directly or indirectly joined. For example configurations where top sheet is directly joined to back sheet by affixing top sheet directly to back sheet, and configurations wherein top sheet is joined to back sheet by affixing top sheet to intermediate members which in turn are affixed to back sheet. Top sheet and back sheet can be affixed directly to each other by attachment means such as an adhesive, sonic bonds, thermal bonds or any other attachment means known in the art. For example, a uniform continuous layer of adhesive, a patterned layer of adhesive, a sprayed pattern of adhesive or an array of separate lines, swirls or spots of construction adhesive may be used to affix top sheet to back sheet. It should be readily appreciated that the above-described attachment means may also be employed to interconnect and assemble together the various other component parts of the article described herein.

The term "backsheet" refers to a material forming a liquid impervious cover of the chassis of the absorbent article. The back sheet prevents the exudates contained in the absorbent core from wetting articles such as bedsheets and overgarments which contact the disposable absorbent article. The back sheet may be a composite layer composed of multiple components assembled side-by-side or laminated. The back sheet may be the same or different in different parts of the chassis. At least in the area of the absorbent core, the back sheet comprises a liquid impervious material in the form of a thin plastic film, e.g., a polyethylene or polypropylene film, a nonwoven material coated with a liquid impervious material, a hydrophobic nonwoven material, which resists liquid penetration, or a laminate of a plastic film and a nonwoven material. The back sheet material may be breathable so as to allow vapour to escape from the absorbent material, while still preventing liquids from passing there through. Examples of breathable back sheet materials are porous polymeric films, nonwoven laminates of spunbond and meltblown layers and laminates of porous polymeric films and nonwoven materials, in such laminates, the polymeric film can have the same or a different width than the nonwoven material. The term "breathable" refers to films having a water vapor transmission rate (WVTR) of at least 300 grams/m² - 24 hours.

As used herein, the "skin-facing" or "bodyside" or " body-facing" or "user facing" or "inner" side or surface means that surface of the article or component which is intended to be disposed toward or placed adjacent to the body of the wearer during ordinary use, while the "outward", "outward-facing", "garment-facing" or "garment" or "outer" side or surface is on the opposite side, and is intended to be disposed to face away from the wearer's body during ordinary use. Such outward surface may be arranged to face toward or placed adjacent to the wearer's undergarments when the absorbent article is worn.

As used herein, the terms "elastic", "elastomeric", "elasticity" or derivations thereof are used to describe the ability of various materials and objects comprised of such to reversibly undergo deformation under stress, e.g., become stretched or extended, in at least one direction when a force is applied to the material and to resume substantially to their original dimensions upon relaxing, i.e., when the force is released, without rupture or breakage. Preferably, it refers to a material or composite which can be elongated in at least one direction by at least 50% of its relaxed length, i.e., elongated to at least 150% of its relaxed length, and which will recover upon release of the applied tension at least 40% of its elongation. Accordingly, upon release of the applied tension at 50% elongation, the material or composite contracts to a relaxed length of not more than 130% of its original length. Examples of suitable elastomer materials include polyether-polyamide block copolymers, polyurethanes, synthetic linear A-B-A and A-B block copolymers, chlorinated rubber/EVA (ethylene-vinyl acetate) blends, EPDM (ethylene-propylene diene monomer) rubbers, EPM (ethylene-propylene monomer) rubbers, blends of EPDM/EPM/EVA, and the like.

As used herein, the term "impermeable" or "impervious" generally refers to articles, chassis and/or elements that are substantially not penetrated by aqueous fluid through the entire thickness thereof under a pressure of 1.0 kPa or less. Preferably, the impermeable article or element is not penetrated by aqueous fluid under pressures of 3.4 kPa or less. More preferably, the impermeable article or element is not penetrated by fluid under pressures of 6.8 kPa or less. An article or element that is not impermeable is permeable.

The term "flat configuration" or "laid-flat state" or "fully stretched state" or "extended state" or "flat-out" configuration is intended to refer to the article when it is flattened into a plane or is substantially flattened into a plane and is used in contrast to when the article otherwise positioned, such as when the article is folded or shaped in or for use by a wearer.

The use of the term "layer" can refer, but is not limited, to any type of substrate, such as a woven web, nonwoven web, films, laminates, composites, elastomeric materials, or the like, or even formulations, such as adhesives, that form a substrate upon a change in conditions (e.g. solidification of a hotmelt adhesive when the temperature drops below a predetermined amount). A layer can be liquid and air permeable, permeable to air but impermeable to liquids, impermeable both to air and liquid, or the like. When used in the singular, it can have the dual meaning of a single element or a plurality of elements.

The term "topsheet" refers to a liquid permeable material sheet forming the inner cover of the chassis of an absorbent article and which in use is placed in direct contact with the skin of the wearer. The topsheet is typically employed to help isolate the wearer's skin from liquids held in the absorbent structure. The top sheet can comprise a nonwoven material, e.g., spunbond, meltblown, carded, hydroentangled, wetlaid or any other type of nonwoven. Suitable nonwoven materials can be composed of man-made fibres, such as polyester, polyethylene, polypropylene, viscose, rayon etc. or natural fibers, such as wood pulp or cotton fibres, hemp, and the like, or other natural or naturally-derived fibers, or from a mixture of two or more natural or man-made fibres. The top sheet material may further be composed of two fibres, which may be bonded to each other in a bonding pattern. Further examples of top sheet materials are porous foams, apertured plastic films, laminates of nonwoven materials and apertured plastic films etc. The materials suited as top sheet materials should be soft and non-irritating to the skin and be readily penetrated by body fluid, e.g., urine or menstrual fluid. The inner coversheet may further be different in different parts of the chassis of an absorbent article. The top sheet fabrics may be composed of a substantially hydrophobic material, and the hydrophobic material may optionally be treated with a surfactant or otherwise processed to impart a desired level of wettability and hydrophilicity.

The term "nonwoven" or "nonwoven fabric or web" means a sheet material having a structure of individual fibers or threads which are interlaid, but not in a regular manner such as occurs with knitting or weaving processes. Nonwoven fabrics or webs have been formed from many processes such as for example, meltblowing processes, spunbonding processes, and bonded carded web processes. Nonwovens may be embossed via an embossing process whereby a pattern is pressed into the fabric, usually by passing the material between rolls with little clearance, and where one or both rolls has a raised design. "Three dimensional embossed nonwovens", also called "embossed nonwovens" may provide enhanced softness and cushioning.

As used herein, an "air-through-bonded" nonwoven, is a nonwoven structure primarily formed by a process that comprises the application of heated air to the surface of the nonwoven fabric. During the through air bonding process, heated air flows through holes in a plenum above the nonwoven material. Unlike hot ovens, which push air through the material, the through air process uses negative pressure of suction to pull the air through an open conveyor apron holding nonwoven as it is drawn through the oven. Pulling air through the material allows the rapid and even transmission of heat to minimize distortion of the nonwoven material. The binding agents used in the through air bonding process include crystalline binder fibers and powders, which melt to form molten droplets throughout the cross-section of the nonwoven. As the material is cooled, bonding occurs at these droplet points.

As used herein, the term "transverse" or "lateral" refers to a line, axis, or direction which lies within the plane of the absorbent article and is generally perpendicular to the longitudinal direction.

Embodiments according to the disclosure will now be described. It is understood that technical features described in one or more embodiments may be combined with one or more other embodiments without departing from the intention of the disclosure and without generalization therefrom.

### THE ABSORBENT ARTICLE

The present disclosure pertains to embodiments of an absorbent article (1), including but not limited to disposable diapers, training pants, adult incontinence products, and feminine hygiene products, designed for the absorption and containment of bodily fluids. The embodiments aim to improve leakage prevention while maintaining wearer comfort and fit at a lowest possible cost.

Absorbent articles herein comprise: a liquid permeable topsheet, a liquid impermeable backsheet, and an absorbent core disposed at least partially between the topsheet and the backsheet. A pair of barrier leg cuffs are included, each comprising an inner cuff element. The inner cuff element comprises an inner folded edge and a plurality of inner elastic elements disposed proximate the inner folded edge, is substantially T-shaped, and comprises a cutaneous sealing strut and an upstanding connecting strut adjoining the cutaneous sealing strut. The upstanding connecting strut is formed by a nonwoven layer, and the cutaneous sealing strut comprises the same nonwoven layer.

Preferably, the same number of inner elastic elements are positioned on either side of the upstanding connecting strut. For example, when an even number of elastics is used e.g. 2 one elastic is positioned on a left side of the upstanding connecting strut and one other of the two elastics is positioned on a right side of the upstanding connecting strut. **In** another example, when an odd number of elastics is used e.g. 3 one elastic is positioned on a left side of the upstanding connecting strut and one other of the three elastics is positioned on a right side of the upstanding connecting strut with the third of the three elastics being positioned substantially inline with the upstanding connecting strut and between the left and right positions of the remaining two elastics. Similarly, when e.g. 5 inner elastic elements are used two will be positioned on the left side of the upstanding connecting strut and two other of the five elastics are positioned on a right side of the upstanding connecting strut with the fifth of the five elastics being positioned substantially inline with the upstanding connecting strut and between the left and right positions of the remaining two elastics, and so on. Advantageously this allows for a substantially symmetrical T-shape for improved comfort and leakage prevention.

Preferably, each barrier leg cuff (5.5') is formed from a single web of material and comprising an inner cuff element (5a, 5a') and an outer cuff element (5b, 5b'); and preferably wherein the outer cuff element (5b, 5b') comprises an outer folded edge (5bf, 5bf') and from 1 to 3 outer elastic elements (7, 7'), wherein the outer elastic elements (7, 7') are disposed proximate the outer folded edge (5bf, 5bf'); and more preferably wherein one or more leg elastics (8, 8') are disposed at least partly between each of the pair of barrier leg cuffs (5, 5') and at least one of the topsheet (2) and the backsheet (3).

The absorbent article (1) is generally constructed around a central chassis, which provides a structural foundation and functional elements for fluid management. The chassis may comprise a liquid permeable topsheet (2), a liquid impermeable backsheet (3), and an absorbent core (4). The topsheet (2) may form the innermost layer (i.e. a skin-facing or body-facing layer), designed for direct contact with the wearer's skin. Its primary function may be to rapidly acquire discharged fluids, such as urine or menstrual fluid, and transport them to the underlying absorbent core (4), while minimizing rewetting and maintaining a dry and comfortable surface against the skin. To achieve this, the topsheet (2) is generally selected to exhibit liquid permeability, softness, and non-irritation. Liquid permeability is achieved through various material properties and structural features, such as pore size and distribution designed for efficient fluid intake, or through capillary action, drawing fluid away from the surface. Surface treatments, in the form of hydrophilic finishes or surfactants, may also be applied to the topsheet (2) to enhance its wettability and liquid acquisition rate. One such preferred example is the use of a zone-treated topsheet with distinct regions of differing wettability. The topsheet (2) may be a nonwoven fabric (e.g., spunbond, meltblown, carded, hydroentangled), an apertured film, or a laminate. Nonwoven fabrics may be composed of synthetic fibers (e.g., polypropylene, polyethylene, polyester), natural fibers (e.g., cotton, cellulose), or combinations thereof. For example, the topsheet (2) may comprise a spunbond nonwoven (e.g. from PFN, 100 Wt% PP). The selection of fiber type and nonwoven construction influences the topsheet's softness, liquid handling properties, and structural integrity. For example, air-through bonded nonwovens are known for softness and loft, while spunbond nonwovens offer good strength and durability.

In a preferred embodiment, the absorbent article comprises a zone-treated topsheet having distinct hydrophilic and hydrophobic regions generally to optimize fluid management and leakage prevention. In this embodiment, a central hydrophilic zone is interposed between two hydrophobic zones positioned proximal and/or adjacent to the cuffs (5, 5'). This configuration in particular helps to addresses a specific challenge often observed with certain topsheet materials, such as those exhibiting enhanced loft, softness, or premium fluid acquisition, which can exhibit an increased propensity for lateral fluid migration. This migration can occur via capillary action within the topsheet structure, guiding fluid underneath the cuff attachment and towards the article's periphery, leading to leakage. By strategically incorporating hydrophobic zones adjacent to the cuff attachment areas, a further barrier is effectively created that impedes this lateral fluid transport, effectively mitigating the risk of side leakage.

The backsheet (3) generally forms the outermost layer (i.e. a garment-facing layer), preventing leakage of fluids from the absorbent core (4) and protecting clothing and bedding. Backsheets herein are preferably in the form of a film. Key characteristics of the backsheet (3) may include liquid impermeability and, optionally, breathability. Liquid impermeability may be achieved through a material that is substantially impermeable to liquids, preventing strike-through even under pressure. Suitable materials include thin plastic films (e.g., polyethylene), coated nonwovens, or laminates of films and nonwovens. The liquid impervious film layer may be made of polyethylene (PE). **In** some embodiments, the backsheet (3) is breathable, allowing water vapor to escape from the absorbent core (4) while maintaining liquid impermeability. This enhances comfort and reduces the risk of skin irritation by promoting airflow. Breathability can be achieved through microporous films or laminates. The backsheet material may be breathable so as to allow vapor to escape from the absorbent material, while still preventing liquids from passing there through. Examples of breathable back sheet materials are porous polymeric films, nonwoven laminates of spunbond and meltblown layers and laminates of porous polymeric films and nonwoven materials, in such laminates, the polymeric film can have the same or a different width than the nonwoven material. The term "breathable" refers to films having a water vapor transmission rate (WVTR) of at least 300 grams/m² - 24 hours. A nonwoven outer cover layer (11) may overlay the backsheet (3) and forms the outer surface of the absorbent article (1). The nonwoven outer cover layer may be a single layer or multi-layer structure.

The absorbent core (4) is generally positioned between the topsheet (2) and backsheet (3) and is the primary component responsible for absorbing and retaining fluids. The absorbent core (4) may be a multi-functional structure designed for efficient fluid management. The absorbent core (4) not only absorbs and stores fluids but may also play a role in distributing fluids and maintaining the integrity of the absorbent article (1). The absorbent core (4) is typically enclosed within a core wrap (12), which is a layer of material (typically a nonwoven) that surrounds the absorbent material. The core wrap generally prevents the absorbent material (e.g., SAP and cellulose fibers) from migrating out of the core and into other parts of the absorbent article (1) and helps to maintain the shape and structural integrity of the absorbent core (4), both when dry and when saturated with fluid. The core wrap material may be a nonwoven fabric, a tissue, or a combination of materials, selected for their strength, flexibility, and fluid handling properties. The absorbent material (13) within the core is generally responsible for absorbing and retaining fluids. This material may be selected from a variety of suitable options, including superabsorbent polymers (SAPs) and cellulose fibers. SAPs are more generally cross-linked polymers capable of absorbing many times their weight in liquid. They may be strategically distributed throughout the absorbent core (4) to maximize fluid retention. Different types of SAP may be selected based on their absorption rate, retention capacity, and gel strength. Cellulose fibers, such as wood pulp fluff, are preferably used in conjunction with SAPs. The cellulose fibers may provide bulk, structure, fluid distribution, and cost-effectiveness. The absorbent material (13) herein preferably comprises a mixture of superabsorbent particles with intermixed and dispersed cellulose fibers therein, for high absorbent capacity and good fluid distribution properties. The absorbent core (4) may further comprise an acquisition and distribution layer, ADL, (14) positioned between the liquid permeable topsheet (2) and the absorbent core (4). The ADL is generally designed to rapidly acquire discharged bodily fluids, such as urine, and then distribute these fluids to the absorbent core (4) for retention. This may help to prevent pooling and rewetting of the topsheet (2), maintaining a dry and comfortable surface against the wearer's skin. The acquisition layer may typically be less hydrophilic than the absorbent layer and may typically be made from a nonwoven, such as a spunbond nonwoven, a carded nonwoven, a through-air bonded, thermo-pressure bonded, or chemically bonded carded nonwoven. The absorbent core (4) may comprise one or more channels (15) that are substantially free of absorbent material. These channels (15) generally facilitate fluid distribution within the absorbent core (4) and prevent bunching or clumping of the absorbent material when the core is wetted. The channels may be embossed but are preferably formed by bonding top and bottom layers of the core wrap together at attachment areas substantially corresponding to the channel shape(s). Exemplary suitable channels are illustrated in Fig.6A-C.

To further prevent the risk of side leakage, the absorbent article (1) includes a pair of barrier leg cuffs (5, 5'). The barrier leg cuffs (5, 5') may be strategically positioned along the longitudinal edges of the chassis to form a seal against the wearer's legs. Each barrier leg cuff (5, 5') is preferably formed from a single web of material and includes an inner cuff element (5a, 5a') and an outer cuff element (5b, 5b'). The primary function of the inner cuff element (5a, 5a') may be to create a close and comfortable seal against the wearer's skin, preventing fluids from escaping the absorbent article (1). The inner cuff element (5a, 5a') generally comprises an inner folded edge (5af, 5af') providing a soft and smooth surface against the wearer's skin, minimizing the risk of irritation. The folded edge may also add bulk and cushioning to the cuff, further enhancing comfort. One or more, preferably a plurality of, inner elastic elements (6, 6') may be disposed proximate the inner folded edge (5af, 5af') to provide a contractive force that gently urges the inner cuff element (5a, 5a') towards the wearer's skin. The use of multiple elastic elements, rather than a single elastic band, may distribute the pressure more evenly, enhancing comfort and reducing the risk of red-marking. The inner cuff element (5a, 5a') may comprise at least 3, preferably from 3 to 5, inner elastic elements (6, 6'). The elastic elements may be secured to the inner cuff element (5a, 5a') using adhesive bonding, thermal bonding, or other suitable methods. The tension of the elastic elements may be carefully controlled to provide an effective seal without causing excessive pressure or discomfort. The outer cuff element (5b, 5b') may provide additional containment and helps to maintain the overall shape and structure of the barrier leg cuff (5, 5'), and importantly may allow the formation of a tight fit with limited formation of wrinkles that would be more prone to leakage. The outer cuff element (5b, 5b') may comprise an outer folded edge (5bf, 5bf') to provide a soft and smooth surface, minimizing the risk of irritation. From 1 to 3 outer elastic elements (7, 7') are disposed proximate the outer folded edge (5bf, 5bf') and contribute to the overall contractive force of the cuff, distributing pressure whilst at the same time reducing risks of pressure marks being created e.g. upon movement of the wearer which may cause inadvertent fold-over of such elastics and/or similar effects resulting from non-optimal size fitting compared to the wearer's body shape. Limiting the number of outer elastic elements may help to prevent such excessive pressure on the skin. While more elastics can spread force, they can also create *multiple localized ridges* or *pressure points,* especially if the strands shift or bunch during wear. Fewer elastics can reduce the number of these ridges, leading to a smoother pressure profile and lower shear on the baby's delicate skin. The number of outer elastic elements provides a balance between reliable leakage protection and a soft and smooth feel, avoiding a too stiff outer cuff. The outer elastic elements (7, 7') may be encapsulated by the outer cuff element (5b, 5b') within an outer fold (f2, f2') thereof to further enhance softness and prevent direct contact of the elastic elements with the skin.

Further one or more leg elastics (8, 8') are preferably disposed directly between each of the pair of barrier leg cuffs (5, 5') and at least one of the topsheet (2) and the backsheet (3) generally to provide a gasketing effect, ensuring a snug fit around the wearer's legs and further preventing leakage. By disposing these further elastic elements between the barrier leg cuffs (5, 5') and the topsheet (2) and/or the backsheet (3), the design ensures maximum contact and conformability to the wearer's body in a cost-effective manner. These leg elastics are generally positioned along the longitudinal edges (9, 9') of the chassis and may extend along only a part of the length of the barrier leg cuffs herein.

It is preferred herein that the one or more leg elastics (8, 8') are disposed directly between each of the pair of barrier leg cuffs (5, 5') and the backsheet (3). Securing the leg elastics (8, 8') directly between the barrier leg cuffs (5, 5') and the backsheet (3), e.g. as opposed to a nonwoven layer like the topsheet, provides several key advantages. First, the lamination strength and overall mechanical integrity of the assembly may be enhanced due to the strong adhesion between the elastics and the film of the backsheet. This configuration offers improved resistance to tearing or delamination, ensuring the leg cuffs maintain their intended position and function throughout the use of the absorbent article (1). Second, this direct securement to the backsheet (3) may ensure that the liquid impermeable film area is positioned as closely as possible to the wearer's skin, indirectly urging the topsheet into closer contact with the skin as well. This intimate contact between the topsheet, backsheet, and skin creates a more effective seal, maximizing the capture and containment of bodily fluids and providing an extra layer of leakage protection. The high securement forces may also ensure that the leg cuffs function as intended throughout the wearing time. For the avoidance of any doubt, the term "directly" in relation to the positioning of the leg elastics (8, 8'), it is understood that the phrase "disposed directly between" is not intended to exclude the presence of adhesive used to secure the leg elastics (8, 8') in their designated location. While the leg elastics (8, 8') are preferably positioned in direct contact with the topsheet (2) and/or backsheet (3), adhesive or other bonding agents may be applied to maintain their position and functionality. The term "directly" is therefore used to indicate that there are no substantial intervening layers of material between the leg elastics (8, 8') and the topsheet (2) and/or backsheet (3), other than suitable amounts of adhesive (or functionally similar alternative) necessary for securement.

To ensure effective gasketing and containment, the absorbent article (1) preferably comprises at least 2 leg elastics (8, 8'), positioned on either (i.e. each) side of the longitudinal axis y typically proximal to lateral edges (also herein referred to as lateral side, longitudinal sides, longitudinal edges, or longitudinal side edges, and generally indicated by reference numerals 9, 9') of the article (sometimes also herein with particular reference to the chassis of the article). The use of multiple leg elastics, and preferably ranging from 2 to 4 leg elastics (generally positioned on each side/lateral-half of the article as mentioned above), distributes the contractive forces more evenly across the leg cuff area, creating a secure and comfortable seal against the wearer's body. This reduces the risk of localized pressure points and enhances the overall fit and conformability of the absorbent article (1), leading to improved leakage prevention.

In addition to the components described above, the absorbent article (1) may incorporate one or more further features designed to enhance its fit, comfort, and functionality. For absorbent articles (1) configured as open diapers, elastic ears may be included at the rear corners of the chassis. These elastic ears provide improved fit and conformability around the wearer's waist and contribute to a more secure and comfortable fit. A fastening system may also be included with open diaper configurations, which enables the securement of the absorbent article (1) around the wearer's body. The fastening system may comprise adhesive tapes, hook-and-loop fasteners, or other suitable mechanical closures. In embodiments utilizing hook-and-loop fasteners, a landing zone or receiving area may be provided on the outer surface of the front portion of the absorbent article (1) to facilitate secure and adjustable fastening. For absorbent articles (1) configured as pull-on pants, elastic front and back waist belts are incorporated into the design. These elastic waist belts are seamed together at the side edges of the absorbent article (1), forming a closed, pant-like configuration. The elastic waist belts provide a snug and comfortable fit around the wearer's waist, allowing the absorbent article (1) to be easily pulled up and down like regular underwear.

### THE BARRIER LEG CUFFS

As exemplified in Fig.2-5, barrier leg cuffs (5, 5') for use herein comprise an inner folded edge (5af, 5af') and a plurality of inner elastic elements (6, 6'), wherein the inner elastic elements (6, 6') are disposed proximate the inner folded edge (5af, 5af'), and preferably wherein the inner elastic elements (6, 6') are encapsulated by the inner cuff element (5a, 5a') within an inner fold (f1, f1'); and, when present, wherein the outer cuff element (5b, 5b') comprises an outer folded edge (5bf, 5bf') and from 1 to 3 outer elastic elements (7, 7'), wherein the outer elastic elements (7, 7') are disposed proximate the outer folded edge (5bf, 5bf'); and preferably wherein the one or more leg elastics (8, 8') are disposed at least partly between each of the pair of barrier leg cuffs (5, 5') and at least one of the topsheet (2) and the backsheet (3). Advantagously, this arrangement creates a multi-layered containment system that effectively prevents leakage and ensures a comfortable, secure fit.

The inner cuff element (5a, 5a'), of barrier cuffs (5, 5, ) herein, is substantially T-shaped; and the inner cuff element (5a, 5a') comprises a cutaneous sealing strut (5cs, 5cs') and an upstanding connecting strut (5us, 5us') adjoining said cutaneous sealing strut (5cs, 5cs'), and wherein the upstanding connecting strut (5us, 5us') is formed by a nonwoven layer, and wherein said cutaneous sealing strut (5cs, 5cs') comprises the same said nonwoven layer. Advantageously, this T-shaped configuration, with a cutaneous sealing strut and nonwoven-formed upstanding connecting strut, provides a superior combination of effective fluid containment and gentle contact with the skin. The T-shape allows the cuff to stand up effectively, while the soft nonwoven material ensures comfort at maximized surface area of contact with the skin for forming an optimised seal yet whilst avoiding risks of pressure marks being formed onto the skin of a wearer.lt is preferred herein that the cutaneous sealing strut (5cs, 5cs') is substantially symmetrical about an axis following a longest dimension of the upstanding connecting strut (5us, 5us'), as for example exemplified in Fig.4 and Fig.5A-C. Typically said longest dimension being substantially perpendicular to the width (w) as described herein. This symmetrical configuration may ensure a balanced distribution of pressure and tension across the skin-contacting area of the cuff, leading to a more comfortable and secure seal. The symmetry helps to prevent the cuff from twisting or buckling, maintaining a consistent and reliable barrier against fluid leakage.

Moreover, as explained above, preferably the the same number of inner elastic elements (6, 6') are positioned on either side of the upstanding connecting strut. Advantageously, this arrangement of elastic elements ensures an equal distribution of contractive forces along the cutaneous sealing strut (5cs, 5cs'), creating a predictable and uniform contact pressure. This symmetry combats any tendency for the cuff to preferentially flex or buckle in one direction, thereby maximizing its ability to adapt dynamically to the wearer's movements and maintain a consistent, gap-free seal, even under conditions of stress or motion.

Each barrier leg cuff (5, 5') herein is preferably constructed from a single, typically continuous, web of material, a design choice offering advantages in manufacturing efficiency and cost-effectiveness. This single-web construction streamlines production by reducing component count and bonding steps. As illustrated for example in Fig. 2-4, the single web material is strategically folded and configured to create both the inner cuff element (5a, 5a') and the outer cuff element (5b, 5b'), thereby generally eliminating separate attachments and/or materials along the cuff's length.

In a preferred advantageous configuration, the outer cuff element (5b, 5b') at least partly overlaps the backsheet (3) on the skin-facing side of the backsheet (3). This arrangement provides several benefits. First, it ensures that the leg cuff, and particularly the outer cuff element (5b, 5b'), is positioned close to the wearer's body, contributing to a more secure and comfortable fit and minimizing gaps that could lead to leakage. Second, this positioning improves the stability and structural integrity of the leg cuff, preventing it from collapsing or folding over during use. Preferably, at least a portion of the outer cuff element (5b, 5b') is disposed directly or indirectly, and more preferably directly, over the backsheet (3). By "directly" here, it is meant that there are no intermediate layers separate from the adhesive and/or the leg elastics (8, 8') themselves as previously defined, so the outer cuff element is directly attached to the backsheet. This direct attachment further enhances the stability and fluid containment capabilities of the leg cuff, while also simplifying the manufacturing process.

In a preferred embodiment, the barrier leg cuffs (5, 5') are constructed from an embossed hydrophobic nonwoven layer. The hydrophobic nature of the material repels fluids, contributing to the overall leakage prevention capabilities of the absorbent article (1). The embossed surface further enhances the functionality and performance of the barrier leg cuffs (5, 5'). The embossments create a three-dimensional texture on the surface of the nonwoven, increasing its loft and providing a softer, more cushion-like feel against the wearer's skin. The embossed patterns may take on various shapes and configurations, such as raised dots, channels, or repeating geometric designs, each contributing to the overall aesthetics and performance of the cuffs. These embossments can be strategically designed to channel fluids, direct airflow, and enhance the overall fit and conformability of the cuffs. The embossments are preferably formed by calendering the nonwoven material with a patterned roll, the roll surface having protrusions of the desired shape of the embossments. This calendering process, which utilizes a combination of pressure and heat, permanently deforms the nonwoven fibers, creating the embossed pattern. This pressure-heat mechanism provides a durable and cost-effective method for creating the desired three-dimensional texture on the barrier leg cuffs (5, 5').

The topographical features imparted to the hydrophobic nonwoven layer of the barrier leg cuffs (5, 5'), achieved through embossing as explained above, represent a deliberate engineering of the material's surface characteristics to optimize both performance and aesthetic attributes. While simplified geometries such as raised circular protuberances and linear channels offer baseline enhancements, the potential extends to more complex and nuanced designs. These may include curvilinear undulations mimicking wave propagation, tessellated hexagonal arrays affording structural integrity and enhanced air permeability, or bio-inspired morphologies such as testudinate (turtle-shell-shaped) embossments exhibiting localized reinforcement and controlled deformation. Such sophistication in surface texturing allows for precise tailoring of the cuff's mechanical properties, modulating its flexibility, conformability, and fluid management behavior. Further, it may be preferred for the nonwoven to comprise differential patterning across the cuff's surface that may enable targeted optimization, accommodating varying functional demands within distinct regions of the barrier leg cuff (5, 5').

In preferred embodiments, the pair of barrier leg cuffs (5, 5') are constructed from a hydrophobic nonwoven material. The use of a hydrophobic material for the barrier leg cuffs (5, 5') enhances their ability to repel fluids, preventing leakage and maintaining a dry and comfortable environment against the wearer's skin. To further optimize performance, the hydrophobic nonwoven may comprise a spunbond nonwoven. Spunbond nonwovens offer a good balance of strength, flexibility, and cost-effectiveness. In particularly advantageous embodiments, the spunbond nonwoven is a lofty spunbond nonwoven comprising crimped fibers. The crimped fibers create a more open and three-dimensional structure, enhancing the loft, softness, and breathability of the barrier leg cuffs (5, 5'). The loftiness provides improved cushioning and conformability, while the increased breathability helps to reduce the risk of skin irritation. Such spunbond nonwovens may be combined with one or more meltblown nonwoven layers to form a spunbond-meltblown nonwoven laminate such as an SM, SMS, SMMS and the like. Particularly when using the mentioned advantageous spunbonds with crimped fibers, combination with a meltblown is particularly advantageous to enhance the nonwoven's ability to form a barrier to liquids whilst retaining the desired softness. **In** case an SM nonwoven layer is opted for, it is arranged such that the spunbond (S) layer is preferably that closest to the skin of the wearer.

Preferably, the outer elastic elements (7, 7') are encapsulated by the outer cuff element (5b, 5b') within an outer fold (f2, f2'). This encapsulation may provide a smoother, more comfortable surface against the wearer's skin, minimizing irritation and chafing. Furthermore, the encapsulation protects the elastic elements from direct contact with bodily fluids, potentially prolonging their lifespan and maintaining their elasticity over time. This protected arrangement also contributes to a cleaner and more aesthetically pleasing appearance of the absorbent article (1).

In embodiments aiming to further enhance leakage protection, the inner cuff element (5a, 5a') is configured to form an up-standing gather. The up-standing gather creates a physical barrier that helps to contain bodily fluids within the absorbent article (1), directing them towards the absorbent core (4) and preventing them from escaping through the leg openings. This feature is particularly effective in managing surges of fluid and preventing side leakage.

To provide a well-defined and reliable containment structure, the outer cuff element (5b, 5b') of each of the pair of barrier leg cuffs (5, 5') is preferably strategically positioned at a lateral edge (9, 9') of the absorbent article (1). This lateral edge (9, 9') extends substantially parallel to the longitudinal axis (y) of the absorbent article (1), providing a continuous barrier along the sides of the product. The lateral edges (9, 9'), in conjunction with a pair of oppositely disposed transversal edges (10, 10') extending substantially perpendicular to the longitudinal axis (y), form a perimeter of the chassis of said absorbent article (1). This configuration ensures a neat and consistent edge definition, contributing to the overall structural integrity and aesthetic appeal of the absorbent article (1), while also providing a reliable barrier against side leakage by extending the cuff to the very edge of the product.

To maximize the effectiveness of the inner cuff element (5a, 5a') in creating a secure and comfortable seal against the wearer's skin, the inner cuff element (5a, 5a') preferably comprises at least 3 inner elastic elements (6, 6'), and preferably ranges from 3 to 5 inner elastic elements (6, 6'). The strategic placement of multiple elastic elements, rather than relying on a single elastic strand, distributes the contractive forces more evenly across the cuff, enhancing its ability to conform to the wearer's body contours and prevent gapping. The increased number of elastic elements also provides redundancy, ensuring that the seal remains effective even if one or more of the elastic elements were to fail or lose tension. The range of 3 to 5 elastic elements balances the need for a secure seal with the desire to maintain a soft and comfortable feel against the skin, avoiding excessive pressure or irritation, and is particularly advantageous in preferred T-shaped cuffs that will be explained in more detail below.

To achieve an optimal balance of containment, comfort, and aesthetic prominence conveying a high performing perception, the inner cuff element (5a, 5a'), such as exemplified in Fig.5A-C, is configured to be substantially T-shaped, preferably when viewed in a cross-section taken about an axis substantially perpendicular to the longitudinal axis (y). This T-shape provides a distinct advantage in conforming to the wearer's body. The vertical portion of the "T" provides height and stand-up rigidity for containing fluids, while the horizontal portion of the "T" provides a wider, softer contact area against the skin, minimizing pressure points and enhancing wearer comfort. This configuration ensures that the inner cuff element (5a, 5a') effectively acts as a fluid barrier without causing undue irritation or discomfort.

To further enhance wearer comfort and minimize the potential for skin irritation, the inner cuff element (5a, 5a') is preferably designed such that its skin-contact surface (5s, 5s') has a defined width (w) when viewed in a cross-section taken about an axis substantially perpendicular to the longitudinal axis (y). This width (w) is preferably greater than the diameter (Ø) of the inner elastic elements (6, 6'). Maintaining a skin-contact surface width (w) that is larger than the diameter (Ø) of the elastic elements ensures that the tensile forces of the elastic elements themselves are dissipated and do not directly point-contact the wearer's skin, which could lead to discomfort or irritation. Preferably, the width (w) is more than 2Ø, more preferably from 3Ø to 150, and even more preferably from 4Ø to 10Ø. These specific ranges for the width (w) provide an optimal balance between distributing the pressure from the elastic elements over a larger area and maintaining a compact cuff profile that does not interfere with the wearer's movements. For the avoidance of doubt, similar benefits are attained in case flat elastics would be used instead of round elastics, in which case the diameter (Ø) would correspond to the width of the flat elastic.

The inner cuff element (5a, 5a') preferably comprises at least two structural components: a cutaneous sealing strut (5cs, 5cs') and an upstanding connecting strut (5us, 5us'). The cutaneous sealing strut (5cs, 5cs') is the portion of the inner cuff element (5a, 5a') that directly contacts the wearer's skin, while the upstanding connecting strut (5us, 5us') adjoins the cutaneous sealing strut (5cs, 5cs') and rises upward, contributing to the overall containment function. The upstanding connecting strut (5us, 5us') is formed by a nonwoven layer, and preferably only one nonwoven layer, to provide a balance of structural support, breathability, and flexibility. This single-layer construction minimizes bulk and stiffness, enhancing wearer comfort and reducing the potential for skin irritation, as well as limiting cost and material usage. The combination of the cutaneous sealing strut (5cs, 5cs') and the upstanding connecting strut (5us, 5us') creates an effective barrier against leakage while maintaining a soft and comfortable feel against the skin.

In a refined embodiment of the above described preferred arrangement, the cutaneous sealing strut (5cs, 5cs'), the portion of the inner cuff element designed for direct skin contact, is constructed with at least two nonwoven layers. These nonwoven layers provide a soft and cushioning effect against the skin, further enhancing wearer comfort and reducing the potential for irritation. The inner elastic elements (6, 6') are then typically disposed between and/or encapsulated within these nonwoven layers. This configuration not only secures the elastic elements in place but also isolates them from direct contact with the skin, preventing any potential discomfort or chafing. Preferably, the at least two nonwoven layers are formed by a single nonwoven layer that is folded around the inner elastic elements (6, 6'). This folded construction simplifies manufacturing and creates a smooth, seamless surface against the skin. Adhesive (16) may be used to join the nonwoven layers together to generally encapsulate the inner elastic elements (6, 6').

To further optimize breathability, flexibility and softness, the cutaneous sealing strut (5cs, 5cs') may comprise one or more adhesive-free gaps (5_{G}) between neighbouring inner elastic elements (6, 6'). These gaps (5_{G}) represent areas where the at least two nonwoven layers are not joined to each other, allowing them to remain separate and promote air circulation within the cuff structure. The presence of these adhesive-free gaps (5_{G}) enhances the overall breathability of the cuff, reducing the risk of moisture buildup and skin irritation. Moreover, without wishing to be bound by theory, the fact that the nonwoven layers are able to move relative to each other in these areas reduce stiffening effects that would otherwise be directly translated onto the skin of the wearer and generated by the tension of the elastics, thus improving the general comfort and softness perception.

To ensure reliable adhesion and positioning of the elastic elements, the inner elastic elements (6, 6') may be strand-coated with adhesive. This strand-coating technique generally applies a thin, uniform layer of adhesive to the elastic elements, providing a strong and durable bond between the elastic elements and the surrounding nonwoven layers, yet permits to minimize the amount of glue by surface area and hence improving on the above mentioned benefits.

The cutaneous sealing strut (5cs, 5cs') may further comprise one or more macro-wrinkles (5_{Mwr}), with each macro-wrinkle preferably substantially corresponding to a position of the one or more adhesive-free gaps (5_{G}). These macro-wrinkles (5_{Mwr}) create a textured surface on the skin-contacting side of the cuff, enhancing softness and flexibility. The macro-wrinkles also help to channel fluids away from the skin, further contributing to comfort, as well as breathability that may be enhanced for a greater dryness perception. The spatial correspondence between the macro-wrinkles (5_{Mwr}) and the adhesive-free gaps (5_{G}) optimizes the balance between breathability, flexibility/softness, and integrity.

In any of the embodiments herein, the absorbent article (1) may be further characterized by specific dimensional relationships between the cutaneous sealing strut (5cs, 5cs') and the upstanding connecting strut (5us, 5us'). **In** such embodiments the cutaneous sealing strut (5cs, 5cs') has a first longest dimension, and the upstanding connecting strut (5us, 5us') has a second longest dimension; and the ratio of the first longest dimension to the second longest dimension is from 0.8 to 2.5, preferably from 0.9 to 2.2, and even more preferably between 1 and 2. Preferably, the first longest dimension runs parallel to the width (w) of the skin-contact surface (5s, 5s') of the inner cuff element (5a, 5a'), and the second longest dimension runs substantially perpendicular thereto. This dimensional relationship contributes to the stability and sealing effectiveness of the barrier leg cuffs (5, 5').

To further refine the performance characteristics of the barrier leg cuffs (5, 5'), specific dimensional parameters of the cutaneous sealing strut (5cs, 5cs') and the upstanding connecting strut (5us, 5us') are preferably implemented. The first longest dimension of the cutaneous sealing strut (5cs, 5cs'), which dictates the contact area with the wearer's skin, is within a range of 5 mm to 30 mm, with a preferred range being 10 mm to 25 mm. This range ensures adequate surface contact for effective sealing against liquid exudates while maintaining flexibility and minimizing skin irritation. Concurrently, the second longest dimension of the upstanding connecting strut (5us, 5us'), which governs the cuff's height and its ability to act as a barrier, is preferably within a range of 6 mm to 30 mm, with a preferred range of 10 mm to 25 mm. This dimensional control ensures sufficient height to contain surges of liquid while preventing the cuff from collapsing or folding over during use. Furthermore, the cutaneous sealing strut (5cs, 5cs') may have a thickness of the cutaneous sealing strut (5cs, 5cs'), ranging from 0.1 mm to 5 mm, preferably between 0.2 mm and 2 mm, provides a balance between conformability to the wearer's anatomy and resistance to compression under load. Finally, the upstanding connecting strut (5us, 5us') may be oriented to be substantially vertical, forming an angle of between 75 and 105 degrees, and more preferably between 85 and 95 degrees, with the topsheet (2). This near-vertical orientation maximizes the cuff's barrier function by presenting a consistent and predictable wall against lateral fluid flow, preventing leakage even under dynamic conditions. This combination of dimensional controls and angular orientation provides superior leakage protection and enhanced wearer comfort.

It is assumed that the present disclosure is not restricted to any form of embodiment described above and that some modifications can be made to the presented embodiments of the invention without departing from the scope of the embodiments as set forth in the claims that follow.

## Claims

1. An absorbent article (1) comprising:
a liquid permeable topsheet (2);
a liquid impermeable backsheet (3);
an absorbent core (4) disposed at least partially between the topsheet (2) and the backsheet (3); and
a pair of barrier leg cuffs (5, 5'), each barrier leg cuff (5.5') comprising an inner cuff element (5a, 5a');
wherein the inner cuff element (5a, 5a') comprises an inner folded edge (5af, 5af') and a plurality of inner elastic elements (6, 6'), wherein the inner elastic elements (6, 6') are disposed proximate the inner folded edge (5af, 5af');
and **characterised in that** the inner cuff element (5a, 5a') is substantially T-shaped; and **in that** the inner cuff element (5a, 5a') comprises a cutaneous sealing strut (5cs, 5cs') and an upstanding connecting strut (5us, 5us') adjoining said cutaneous sealing strut (5cs, 5cs'), and preferably wherein the upstanding connecting strut (5us, 5us') is formed by a nonwoven layer, and wherein said cutaneous sealing strut (5cs, 5cs') comprises the same said nonwoven layer.

2. An absorbent article according to Claim 1 wherein each barrier leg cuff (5.5') is formed from a single web of material and each said cuff comprising an inner cuff element (5a, 5a') and an outer cuff element (5b, 5b'), preferably wherein the outer cuff element (5b, 5b') comprises an outer folded edge (5bf, 5bf') and from 1 to 3 outer elastic elements (7, 7'), wherein the outer elastic elements (7, 7') are disposed proximate the outer folded edge (5bf, 5bf'), and more preferably one or more leg elastics (8, 8') are disposed at least partly between each of the pair of barrier leg cuffs (5, 5') and at least one of the topsheet (2) and the backsheet (3).

3. An absorbent article according to Claim 2 wherein the outer elastic elements (7, 7') are encapsulated by the outer cuff element (5b, 5b') within an outer fold (f2, f2') thereof.

4. An absorbent article according to any of Claims 2-3 wherein the one or more leg elastics (8, 8') are disposed at least partly between each of the pair of barrier leg cuffs (5, 5') and the backsheet (3).

5. An absorbent article according to any of Claims 2-4 wherein the article comprises at least 2 of said leg elastics (8, 8'), preferably from 2 to 4 of said leg elastics.

6. An absorbent article according to any of Claims 2-5 wherein the inner cuff element (5a, 5a') forms an up-standing gather.

7. An absorbent article according to any of Claims 2-6 wherein the outer cuff element (5b, 5b') of each of the pair of barrier leg cuffs (5, 5') is positioned at a lateral edge (9, 9') of said article, said lateral edge (9, 9') extending substantially parallel to a longitudinal axis (y) and preferably wherein a pair of said lateral edges (9, 9') together with a pair of oppositely disposed transversal edges (10, 10') extending substantially perpendicular to the longitudinal axis (y) form a perimeter of a chassis of said article (1).

8. An absorbent article according to any of the preceding Claims wherein the inner cuff element (5a, 5a') comprises at least 3, preferably from 3 to 5, inner elastic elements (6, 6').

9. An absorbent article according to any of the preceding Claims wherein the inner cuff element (5a, 5a') comprises a skin-contact surface (5s, 5s') having a width (w) when viewed from a section taken about an axis being substantially perpendicular to the longitudinal axis (y), and wherein said width (w) is greater than a diameter (Ø) of inner elastic elements (6, 6'); preferably wherein said width (w) is more than 2Ø, more preferably from 3Ø to 15Ø, even more preferably from 4Ø to 10Ø.

10. An absorbent article according to any of the preceding Claims wherein the inner cuff element (5a, 5a') comprises a cutaneous sealing strut (5cs, 5cs') and an upstanding connecting strut (5us, 5us') adjoining said cutaneous sealing strut (5cs, 5cs'), and wherein the upstanding connecting strut (5us, 5us') is formed by a nonwoven layeronly one of said nonwoven layer.

11. An absorbent article according to any of the preceding Claims wherein the cutaneous sealing strut (5cs, 5cs') comprises at least two nonwoven layers and having said inner elastic elements (6, 6') disposed therebetween and/or encapsulated therein, preferably said at least two nonwoven layers being formed by a single nonwoven layer folded around said inner elastic elements (6, 6').

12. An absorbent article according to any of the preceding Claims wherein the cutaneous sealing strut (5cs, 5cs') comprises one or more adhesive-free gaps (5G) between neighbouring inner elastic elements (6, 6') preferably such that the at least two nonwoven layers are not joined to each other at positions(s) corresponding to said gap(s) and are able to stay apart.

13. An absorbent article according to any of the preceding Claims wherein the inner elastic elements (6, 6') are strand-coated with adhesive.

14. An absorbent article according to any of the preceding Claims wherein the cutaneous sealing strut (5cs, 5cs') comprises one or more macro-wrinkles (5Mwr), each macro-wrinkle preferably substantially corresponding to a position of the one or more adhesive-free gaps (5G).

15. An absorbent article according to any of the preceding Claims wherein the pair of barrier leg cuffs (5, 5') comprise a hydrophobic nonwoven, preferably wherein the hydrophobic nonwoven comprises a spunbond nonwoven, even more preferably wherein the spunbond nonwoven is a lofty spunbond nonwoven comprising crimped fibers.

16. An absorbent article according to any of Claims 2-15 wherein the one or more leg elastics (8, 8') are disposed directly between each of the pair of barrier leg cuffs (5, 5') and at least one of the topsheet (2) and the backsheet (3), preferably wherein the one or more leg elastics (8, 8') are disposed directly between each of the pair of barrier leg cuffs (5, 5') and the backsheet (3).

17. An absorbent article according to any of Claims 2-16 wherein the outer cuff element (5b, 5b') at least partly overlaps the backsheet (3) on the skin-facing side of the backsheet (3), and preferably wherein at least a portion of the outer cuff element (5b, 5b') is disposed directly or indirectly, preferably directly, over the backsheet (3).

18. An absorbent article according to any of the preceding Claims wherein the cutaneous sealing strut (5cs, 5cs') has a first longest dimension, and the upstanding connecting strut (5us, 5us') has a second longest dimension; and wherein a ratio of first longest dimension/second longest dimension is from 0.8 to 2.5, preferably from 0.9 to 2.2, even more preferably between 1 and 2; preferably wherein the first longest dimension runs parallel to a width (w) of a skin-contact surface (5s, 5s') and the second longest dimension runs substantially perpendicular thereto.

19. An absorbent article according to any of the preceding Claims wherein the cutaneous sealing strut (5cs, 5cs') is substantially symmetrical about an axis following a longest dimension of the upstanding connecting strut (5us, 5us'); and/or wherein the the same number of inner elastic elements (6, 6') are positioned on either side of the upstanding connecting strut 5us, 5us').
